# EUROPEAN PATENT APPLICATION

(11) **EP 0 738 516 A1**
(43) Date of publication of application: **23.10.1996**
(21) Application number: 95936753.3
(22) Date of filing: 08.11.1995
(51) Int. Cl.: A61K 38/57, A61K 7/40, A61K 9/06, A61K 9/08

(54) **EXTERNAL PREPARATION FOR SKIN PROTECTION**

(30) Priority: 08.11.1994 JP 274073/94
(71) Applicant: MOCHIDA PHARMACEUTICAL CO., LTD., Shinjuku-ku Tokyo 160 (JP)
(72) Inventor: TERAO, Toshihiko, Shizuoka-ken 431-31 (JP); KANAYAMA, Naohiro, Shizuoka-ken 431-31 (JP)
(74) Representative: Grünecker, Kinkeldey, Stockmair & Schwanhäusser Anwaltssozietät
(86) International application number: JP9502282
(87) International publication number: WO9614085

(57) **Abstract**

The present invention relates to a dermatological agent for skin protection characterized by its inclusion of ulinastatin which is effective in preventing skin dryness, skin keratosis and skin aging, and in treating acne vulgaris and atopic dermatitis, and the like.

The dermatological agent for skin protection of the present invention may be used for prevention and improvement of skin troubles such as skin dryness, skin keratosis, skin aging and dandruff; improvement of moisture retaining properties of the skin; and prevention and treatment of acne vulgaris and its sequela, and atopic dermatitis and itching associated with the atopic dermatitis. The dermatological agent for skin protection of the present invention is highly safe.

## Description

### TECHNICAL FIELD

The present invention relates to a dermatological agent for skin protection characterized by its inclusion of ulinastatin. More specifically, the present invention relates to an agent for preventing skin dryness, an agent for treating skin keratosis, an agent for preventing skin aging, a skin humectant, an agent for treating acne vulgaris, and an agent for treating atopic dermatitis. The terms, "treating agent" and "preventing agent" used herein include prophylactic. The dermatological agent of the present invention is directed to a drug, a quasi-drug, or a cosmetic.

### BACKGROUND TECHNOLOGY

Ulinastatin (hereinafter referred to as UTI) is a tripsin inhibitor of human urine origin, and its use for drugs has been investigated in many publications (Japanese Patent Application Laid-Open No. 51-123810 (anti-inflammatory injection), Japanese Patent Application Laid-Open No. 55-160724 (anti-shock agent), Japanese Patent Application Laid-Open No. 57-144224 (therapeutic agent for respiratory organ complaints), Japanese Patent Application Laid-Open No. 58-225026 (prophylactic and therapeutic agent for diseases caused by sthenia of amylase activity and/or lipase activity), Japanese Patent Application Laid-Open No. 62-205033 (therapeutic agent for rheumatoid arthritis), Japanese Patent Application Laid-Open No. 63-267730 (topical agent for treating allergic diseases), Japanese Patent Application Laid-Open No. 1-203334 (agent for relief of the side effects induced by platinum-containing drugs), Japanese Patent Application Laid-Open No. 4-117331 (therapeutic agents for hepatitis), and the like). Such investigations were mainly directed to systemic administration, for example, by intravenous or oral administration, and some topical dosage forms for administration through mucous membrane such as opthalmic and sollunarium were also known. Preparation for external use including the UTI was also known wherein the UTI is used for treating and preventing AIDS (Japanese Patent Application Laid-Open No. 2-145527). There has been no report, however, where direct action of the UTI on the skin itself has been investigated, and the skin protecting action of the substance was unknown.

In the external preparations for skin protection, various ingredients extracted from natural materials, for example, extracts of animal or vegetable origin, polysaccharide, protein, natural high polymer, and the like have been blended for cosmetic purpose for instance, in view of their characteristic actions upon their use. Their actions, however, were not necessarily sufficient, and there has been some risk of inducing atopic dermatitis, whose patient have rapidly increased in these days, when they are used in infants, children, those who have sensitive skin, or women since many such ingredients are foreign to human. In view of such situation, development of a skin protective agent which may be used for a prolonged period with high safety and effectivity has been awaited.

### SUMMARY OF THE INVENTION

The inventors of the present invention were interested in the fact that fetus and new-born infant within several months from their birth have a high skin moisture-retaining ability and exhibit low occurrence of skin disorder, and the inventors paid attention to the UTI in amniotic fluid, and action of the UTI on the skin was investigated in detail. As a result of such investigation, the inventors found that UTI is provided with various skin protecting action including prevention of skin dryness, prevention of skin keratinization, prevention of skin aging, improvement of skin moisture retaining ability, and improvement of acne vulgaris. The inventors of the present invention made further studies on the bases of the above-described findings to thereby solve the problems as described above, and the present invention has been completed after such efforts.

### BEST MODE FOR CARRYING OUT THE INVENTION

Next, the constitution of the present invention is described.

The dermatological agent for skin protection of the present invention is characterized by its inclusion of the UTI, and is used, for example, as a drug, a quasi-drug, or a cosmetic for the purpose of at least one of prevention of skin troubles such as skin dryness, keratosis, aging and dandruff; treatment of itching associated with such conditions; improvement of moisture retaining properties of the skin; prevention and treatment of acne vulgaris and its sequela; treatment of stiffness, tension, itching and the like at excoriation including acne; and prevention and treatment of atopic dermatitis and itching associated with the atopic dermatitis. It should be noted that mucous membrane regions such as those in eyes and nose are excluded from the terminology of the term, skin.

In addition, the dermatological agent for skin protection of the present invention is free from side effects and safe, and also less stimulative, and therefore, the agent can be preferably used in the above-described purpose for women with sensitive skin, those who are likely to suffer from atopic dermatitis, children and infants, and in particular, for children and infants, in addition to healthy normals.

The dermatological agent for skin protection of the present invention may be formulated in any dosage form, and exemplary dosage forms include solubilized dosage forms (e.g. liquid dosage form, lotion, and gel liquid), emulsion dosage forms (e.g. emulsion ointment, cream, and milky lotion), water-soluble ointment, water phase/oil phase-double phase dosage form, spray, paste, and liniment.

The content of UTI in the dermatological agent for skin protection of the present invention may differ depending on the dosage form of the dermatological agent. The content, however, may preferably be from 0.001 to 10% by weight, and more preferably, from 0.01 to 5% of the total weight of the formulation. Content in excess of 10% by weight is pharmaceutically unpreferable, and economically nonadvantageous.

The dermatological agent for skin protection of the present invention may optionally include various components generally used in drugs, quasi-drugs, and cosmetics in addition to the UTI, which is the essential component, at a content that would not adversely affect the merits of the present invention. Exemplary such components include aqueous components (water, alcohol water etc.), humectant (e.g. propylene glycol, polyethylene glycol, glycerin, etc.), thickener (e.g. sodium arginate, gum arabic, carboxyvinyl polymer, carboxymethyl cellulose, etc.), surfactant (e.g. glycerine isostearate, polyoxyethylene hydrogenated castor oil, etc.), UV absorbent (e.g. 2-hydroxy-4-methoxy benzophenone, Ubinal 490, etc.). antiseptics (e.g. paraben, methylparaben, benzethonium chloride, chlorhexidine, etc.), antioxidants (e.g. BHT, ascorbic acid, α-tocopherol, etc.), skin penetrant (e.g. quaternary ammonium salts such as benzalkonium chloride, and epoxidated nonyl phenol, etc.), colorants (e.g. inorganic pigments such as iron oxide and chromium oxide, dyes such as rhodamine B and β-carotene), and other pharmaceutically effective components; and general additives such as perfume.

The present invention is also directed to a method of skin protection utilizing UTI, and more specifically, to a method for prevention of skin troubles such as skin dryness, keratosis, aging and dandruff; treatment of itching associated with such conditions; improvement of moisture retaining properties of the skin; treatment of acne vulgaris and its sequela; treatment of stiffness, tension, itching and the like at excoriation including acne; and prevention and treatment of atopic dermatitis and itching associated with the atopic dermatitis.

The present invention is also directed to a method for producing a medical composition which is effective in preventing and/or treating the above-mentioned pathological conditions by combining the ulinastatin, which is the effective component, with a pharmaceutically acceptable carrier.

The present invention is also directed to a use of ulinastatin for the production of a preparation used for prevention of skin troubles such as skin dryness, keratosis, aging and dandruff; treatment of itching associated with such conditions; improvement of moisture retaining properties of the skin; treatment of acne vulgaris and its sequela; treatment of stiffness, tension, itching and the like at excoriation including acne; and prevention and treatment of atopic dermatitis and itching associated with the atopic dermatitis.

The present invention is described in detail by referring to the Examples and Experiments, which by no means limit the scope of the present invention.

### (1) Protection effects for dry skin

In 100 ml of sterilized distilled water was dissolved 50,000 units of UTI to prepare UTI solution (500 U/ml).

Effects of continuous application for one week to three months were investigated in eight cases of female patients of 20 to 30 years old with subjective and objective symptom of dry skin (with no complication). The UTI solution was applied on the entire area of the face twice a day after washing the face in the morning and in the evening, and the patients recorded the degree of skin dryness in their symptom diary every day. With regard to objective symptom, a physician examined the skin condition with naked eye, and an close-up photograph of the skin on the right cheek of the patient was taken before the application, and after the application from the second day as day passed by, for three months at maximum. The history was thus observed.

The results are shown in Table 1.

**Table 1**

| Effects of application of UTI solution (500 U/ml) | | | | | | |
|---|---|---|---|---|---|---|
| Application period | Before application | One day | One week | Two weeks | Four weeks | Three months |
| | | | | | | |

| Subjective symptom | | | | | | |
|---|---|---|---|---|---|---|
| Skin dryness | 8/8 | 0/8 | 0/8 | 0/6 | 0/4 | 0/2 |
| Skin smoothness | 0/8 | 1/8 | 7/8 | 6/6 | 4/4 | 2/2 |

| Objective symptom | | | | | | |
|---|---|---|---|---|---|---|
| Skin gloss (luster) | 0/8 | 2/8 | 6/8 | 6/6 | 4/4 | 2/2 |

In all cases, the patient reported disappearance of the symptom of the skin dryness on the next day of the application, and the effect was maintained throughout the test period. The skin smoothness (glistening) was noticed in almost all the cases in approximately three days, and in seven cases out of eight cases after one week. With regard to the objective symptom, the skin gloss (luster) was noticed in six cases out of eight cases after one week.

In one of the cases investigated, moisture content was measured with a moisture measurement system (IM-3SCV manufactured by Fuji-Technica), and the value of 3600 OD before the application increased to 3750 OD by the application for one week, indicating the improvement of the moisture retaining ability of the skin. The measurement was carried out in accordance with the method of Terao et al. (Jpn. Soc. Med. Electronics. Biol. Eng. vol. 31, page 377, 1993).

UTI solution of 5000 U/ml was applied in another two cases, and UTI solution of 50 U/ml was applied in the additional three cases, and the progress was observed. The results are shown in Tables 2 and 3.

**Table 2**

| Effects of application of UTI solution (5000 U/ml) | | | | | |
|---|---|---|---|---|---|
| Application period | Before application | One day | One week | Two weeks | Four weeks |
| | | | | | |

| Subjective symptom | | | | | |
|---|---|---|---|---|---|
| Skin dryness | 2/2 | 0/2 | 0/2 | 0/2 | 0/2 |
| Skin smoothness | 0/2 | 1/2 | 2/2 | 2/2 | 2/2 |

| Objective symptom | | | | | |
|---|---|---|---|---|---|
| Skin gloss (luster) | 0/2 | 1/2 | 2/2 | 2/2 | 2/2 |

In the cases where the 5000 U/ml solution was applied, both the subjective symptom and the objective symptom were similar to those of the cases wherein the 500 U/ml solution was applied.

**Table 3**

| Effects of application of UTI solution (50 U/ml) | | | | | |
|---|---|---|---|---|---|
| Application period | Before application | One day | One week | Two weeks | Four weeks |
| | | | | | |

| Subjective symptom | | | | | |
|---|---|---|---|---|---|
| Skin dryness | 3/3 | 2/3 | 2/3 | 0/3 | 0/3 |
| Skin smoothness | 0/3 | 0/3 | 1/3 | 2/3 | 2/3 |

| Objective symptom | | | | | |
|---|---|---|---|---|---|
| Skin gloss (luster) | 0/3 | 0/3 | 1/3 | 2/3 | 2/3 |

In the cases where the 50 U/ml solution was applied, the effect of the application was less significant than the cases wherein the 500 U/ml solution was applied. Accordingly, the dermatological agent for skin protection of the present invention has strong moisture-retaining effects for human dry skin, and contributes for the disappearance of the feeling of skin dryness and the recovery of the feeling of skin smoothness.

### (2) Effect of stabilizing skin keratinization cell (fibroblasts)

To the preliminarily cultivated human fibroblasts (of vagina origin) was added Fura2-AM (5 µg) (37°C, 5% CO₂ incubator). UTI was then added to the culture medium (100 U/ml), and preincubated at 23°C for 70 minutes. This group was designated group A, and the group wherein physiological saline was added instead of the UTI was designated group B. lipopolysaccharide (LPS) (10 µg/ml) was added to both of the group A and the group B after the preincubation, and the amount of Ca²⁺ in the fibroblasts was observed.

A rapid increase in Ca²⁺ in the fibroblasts occurred in the group with no addition of UTI, while such increase of Ca²⁺ in the fibroblasts was not at all observed in the group where UTI had been added. It was also found that 100 U/ml of UTI is sufficient for suppressing the increase of Ca²⁺ in the fibroblasts induced by the LPS.

The results as described above confirmed the fibroblast stabilization effect of the UTI, and in particular, stabilization effect of the UTI for foreign stimulant, and UTI was thus estimated to be effective in preventing excessive activation of the fibroblast, namely, keratinization and aging of the skin; alteration of exfoliation property of the skin; and treatment of dandruff.

### (3) Effect for acne vulgaris

To 100 ml of sterilized distilled water was dissolved 50,000 units of UTI to prepare a UTI solution. In seven trial subjects (six females and one male) of 16 to 26 years old suffering from acne vulgaris, UTI solution was continuously applied twice a day in the morning and in the evening for four weeks to investigate the effect of the UTI solution for the acne, and in particular, the effects of the UTI solution for flare, pustula, induration and pigmentation associated with the acne. The results of the judgment by the physician are shown in Table 4.

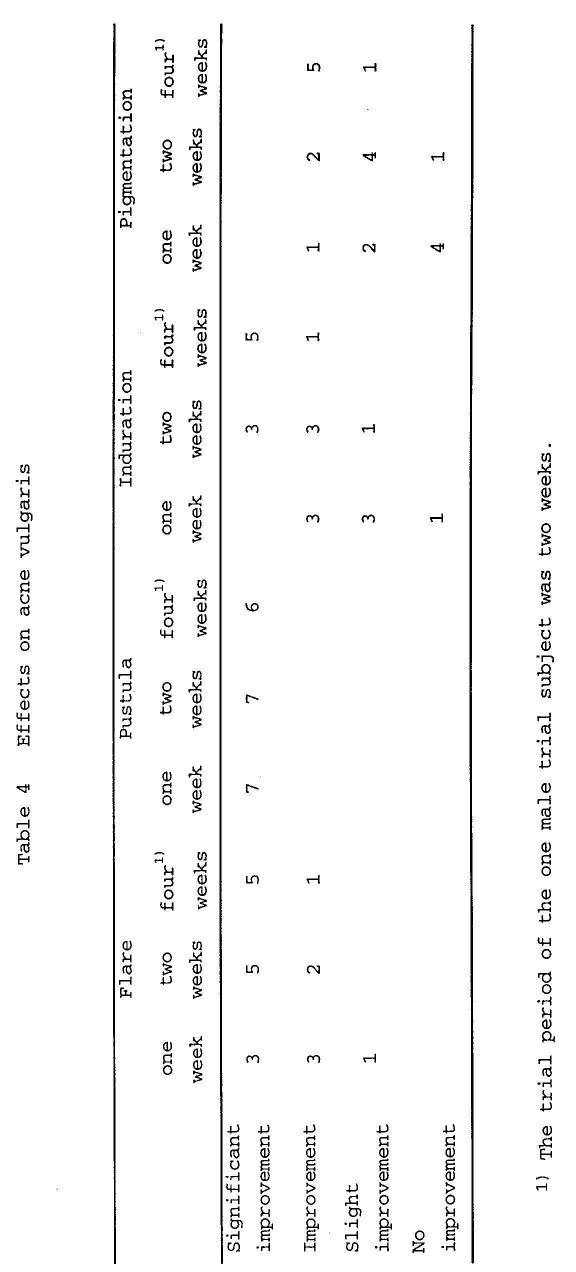

As shown in the results, improvement was demonstrated in all of the flare, the pustura, the induration and the pigmentation. UTI solution exhibited particularly significant effects on acute symptoms such as flare and pustura. The pigmentation, which is a serious sequela of acne, was able to be substantially prevented by the use of aqueous UTI solution, and a clear improvement in the existing pigmentation was also recognized in the case wherein the UTI solution was used for 4 weeks or over.

UTI was tested in another four cases after mixing with commercially available lotion and milky lotion, respectively, and significant improvements were observed in all the cases.

### (4) Effect for atopic dermatitis

In three female and three male subjects of 14 to 27 years old who have been diagnosed as atopic dermatitis, UTI solution (100 U/ml) was applied on the sites of eczema on their faces or limbs everyday for two months, and confirmation of their subjective symptoms and observation of the affected lesion was carried out as time passed by. The results of the judgment by the physician are shown in Table 5.

**Table 5**

| Effects for atopic dermatitis | | | | | | |
|---|---|---|---|---|---|---|
| | (Itching) | | (Flare) | | (Eczema size) | |
| | one month | two months | one month | two months | one month | two months |
| Significant improvement | 5 | 6 | | | 1 | 1 |
| Improvement | | | 2 | 4 | | 2 |
| Slight improvement | 1 | | 4 | 2 | 3 | 3 |
| No improvement | | | | | 2 | |

Disappearance of the itching, and size reduction of the flare and the eczema were observed with lapse of time to demonstrate the excellent effect of the UTI for atopic dermatitis. With regard to the itching, the symptom disappeared in all cases in two months, demonstrating the excellent effect of the UTI.

UTI solution (500 U/ml) was tested on another two patients of atopic dermatitis to evaluate the effects. The UTI solution was continuously applied on the sites of eczema on their faces or limbs. Improvements in subjective symptoms such as itching as well as improvements in erythema, weep and pimples were found after one week, and the improvements were even more significant after two weeks.

Improvements in the feeling of skin dryness and recovery of the feeling of skin smoothness were also recognized in these cases of atopic dermatitis.

It should be noted that no side effects were recognized in the use of the UTI in the above (1) to (4).

### EXAMPLES

Next, the examples of the preparations of the present invention is described.

### (Example 1) Gel ointment (0.1% preparation)

| | |
|---|---|
| UTI ingredient (126900 U/ml) | 2.13 ml |
| carboxyvinyl polymer | 1.2 g |
| diisopropanolamine | 0.3 g |
| ethanol | 38 g |
| purified water | 58.37 g |

Carboxyvinyl polymer was dispersed in the mixture of ethanol and purified water by incrementally adding the carboxyvinyl polymer into the mixture with stirring. UTI ingredient was added and dispersed in the mixture. Diisopropanol amine was added dropwise to the mixture to prepare a gel ointment.

### (Example 2) Liquid dosage form

50,000 units of UTI was dissolved in 10 ml, 100 ml, 500 ml, and 1000 ml of sterilized distilled water, respectively, to prepare UTI liquid dosage form (5000 U/ml, 500 U/ml, 100 U/ml, and 50 U/ml).

### (Example 3) Ointment A (0.1% preparation)

| | |
|---|---|
| UTI ingredient (126900 U/ml) | 2.13 ml |
| isopropyl myristate | 5 g |
| liquid paraffin | 5 g |
| cetanol | 6 g |
| glyceryl monostearate | 10 g |
| polyoxyethylene cetyl ether | 3 g |
| propylparaben | 0.1 g |
| methylparaben | 0.1 g |
| purified water | 68.67 g |

The components as described above were mixed by a conventional procedure to prepare 100 g ointment preparation.

### (Example 4) Ointment B (0.1% preparation)

| | |
|---|---|
| UTI ingredient (126900 U/ml) | 2.13 ml |
| white soft paraffine | 25 g |
| stearyl alcohol | 20 g |
| propylene glycol | 12 g |
| polyoxyethylene hydrogenated castor oil | 4 g |
| glyceryl monostearate | 6 g |
| propylparaben | 0.1 g |
| methylparaben | 0.1 g |
| purified water | 37.00 g |

The components as described above were mixed by a conventional procedure to prepare 100 g ointment preparation.

### (Example 5) Ointment C (0.1% preparation)

| | |
|---|---|
| UTI ingredient (126900 U/ml) | 2.13 ml |
| polyoxyl 40 stearate | 2 g |
| glyceryl monostearate | 5 g |
| cetanol | 5 g |
| liquid paraffin | 8 g |
| middle-chain fatty acid triglyceride | 8 g |
| propylene glycol | 8 g |
| propylparaben | 0.1 g |
| methylparaben | 0.1 g |
| purified water | 61.67 g |

The components as described above were mixed by a conventional procedure to prepare 100 g ointment preparation.

### (Example 6) Ointment D (1% preparation)

| | |
|---|---|
| UTI ingredient (126900 U/ml) | 21.3 ml |
| isopropyl myristate | 5 g |
| liquid paraffin | 5 g |
| cetanol | 6 g |
| glyceryl monostearate | 10 g |
| polyoxyethlene cetyl ether | 3 g |
| propylparaben | 0.1 g |
| methylparaben | 0.1 g |
| purified water | 49.5 g |

The components as described above were mixed by a conventional procedure to prepare 100 g ointment preparation.

### (Example 7) Liquid dosage form

Lyophilized UTI was dissolved in lotion (Collage®, Mochida Pharmaceutical Co., LTD.) to prepare a liquid dosage form with a UTI concentration of 5000 U/ml.

### INDUSTRIAL UTILITY

The dermatological agent for skin protection of the present invention may be used for prevention of skin troubles such as skin dryness, keratosis, aging and dandruff; treatment of itching associated with such conditions; improvement of moisture retaining properties of the skin; treatment of acne vulgaris and its sequela; treatment of stiffness, tension, itching and the like at excoriation including acne; and prevention and treatment of atopic dermatitis and itching associated with the atopic dermatitis. The external preparation of the present invention is a highly safe dermatological agent for skin protection that may be used for women with sensitive skin, those who are likely to suffer from atopic dermatitis, children and infants in addition to healthy normals.

## Claims

1. A dermatological agent for skin protection characterized in that said agent contains ulinastatin.

2. The dermatological agent according to claim 1 wherein said agent is an agent for preventing skin dryness.

3. The dermatological agent according to claim 1 wherein said agent is an agent for treating skin keratosis.

4. The dermatological agent according to claim 1 wherein said agent is an agent for preventing skin aging.

5. The dermatological agent according to claim 1 wherein said agent is an agent for treating acne vulgaris.

6. The dermatological agent according to claim 1 wherein said agent is an agent for treating atopic dermatitis.

7. The dermatological agent according to claim 1 wherein said agent is an agent for treating itching associated with atopic dermatitis.

8. The dermatological agent according to any one of claims 1 to 7 in a dosage form of cream.

9. The dermatological agent according to any one of claims 1 to 7 in liquid dosage form.

10. The dermatological agent according to any one of claims 1 to 7 in a dosage form of lotion.
